Europäisches Patentamt

European Patent Office

Office européen des brevets

(11) Publication number : **0 483 043 A2**

(12) # EUROPEAN PATENT APPLICATION

(21) Application number : **91510002.8**

(22) Date of filing : **24.10.91**

(51) Int. Cl.⁵ : **G01N 9/00,** G01N 33/42

(30) Priority : **24.10.90 ES 9002698**

(43) Date of publication of application :
**29.04.92 Bulletin 92/18**

(84) Designated Contracting States :
**AT BE CH DE DK ES FR GB GR IT LI LU NL SE**

(71) Applicant : **ASFALTOS ESPANOLES, S.A. (ASESA)**
**Autovia de Salou, s.n.**
**E-43006 Tarragona (ES)**

(72) Inventor : **Torres Estrada, Josep**
**Prat de la Riba, 24**
**E-43001 Tarragona (ES)**
Inventor : **Ramos Triguero, Alfonso**
**Rambla Vella, 18**
**E-43003 Tarragona (ES)**
Inventor : **Gonzalez Escoda, Josep Ma**
**Gasometre, 20**
**E-43001 Tarragona (ES)**
Inventor : **Peralta Huguet, Xavier**
**Pons Icart, 23**
**E-43004 Tarragona (ES)**

(74) Representative : **Ferregüela Colon, Eduardo**
**Agricultura, 99**
**E-08019 Barcelona (ES)**

(54) **Feeder device of samples for vibrating tube electronic densimeters.**

(57) A sample feed device for vibrating tube electronic densimeters, of the type comprising a measuring cell (1), provided with a charge port (2) and a discharge port (3). The device comprises a heat-insulated block (4) provided with an upper port (5) and a lower port (8) communicating over passages (6) and (9) with charge and discharge (10) ports capable of being engaged with the corresponding ports (2) and (3), there being provided a suction chamber (11) having an inlet port (12) connected to the port (8) and an open end (13), fitted to a collector container (14), provided with a suction port (15) connected to suction means (16). The block (4) and the cell (1) are held at a constant temperature throughout the test.

Applicable to the determination of the density of highly viscous products such as bitumens.

FIG. 3

EP 0 483 043 A2

## Field of the Invention

The present invention relates to a sample feed device for vibrating tube electronic densimeters, of the type particularly applicable to the determination of the density of high viscosity materials, such as for example bituminous materials, and more precisely of high density materials such as bitumens.

The invention may be used for all those products having a viscosity allowing them to be pumped at the operating temperature and pressure.

## Prior Art

Up to now, the method used almost exclusively for the determination of the density of bituminous products and more particularly of bitumens was based on ascertaining the relative density by way of a pyknometer, which was obtained by comparing the masses of like volumes of material and water at one same temperature.

This method is very slow and time consuming, at the same time as it involves a relatively high error risk, due to the many operations which have to be performed and which extend the test over 3 to 4 hours.

There has recently been put into practice a relatively novel way of determining the density of fluid products, based on a principle of physics, according to which when a mass of a particular volume starts to vibrate at a constant temperature which avoids variations of said volume, the frequency of vibration depends on the density of the said mass, whereby, by measuring the frequency, the density of the vibrating mass may be deduced.

The densimeters allowing this new method to be practised are vibrating tube electronic densimeters, comprising an essentially U-shaped measuring cell, in which the sample whose density is to be determined is introduced. Said sample must be homogenous and free of air bubbles.

The sample is introduced under pressure in the measuring cell, for example by means of a piston pump or peristaltic pump. Nevertheless, when determining viscous products, such as asphalts and bitumens, it is extremely hard to introduce the sample and even when it is managed to introduce a homogenous sample, it is practically impossible to remove it. Furthermore, when removal of the sample is achieved, the measuring cell must be carefully cleaned, which is a very arduous task.

## Summary of the Invention

Against all the drawbacks of the present state of the art mentioned, the device of the present invention allows the sample to be introduced and removed and the measuring cell then to be cleaned with great ease and speed and, thanks thereto, the density of a product such as a bitumen may be very easily and accurately determined in a time of about two minutes.

In essence, the device in question is characterized in that it comprises a heat-insulated block provided with an upper sample inlet port, communicating over a first internal passage with a charge port and a lower outlet port, communicating with a discharge port over a second internal passage, said charge and discharge ports of the heat-insulated block being so disposed that in the operative position of the feed device they are respectively firmly engaged with the corresponding charge and discharge ports of the measuring cell, there being disposed below the block a suction chamber provided with an upper inlet port, connected to the lower outlet port of the block, having a lower mouth adapted to be sealingly attached to the mouth of a waste collector container in which the sample falls after the density thereof has been determined, and a suction port, adapted to be connected to a vacuum or suction pump, the arrangement being such that the sample is introduced in the measuring cell by suction thereof, due to the negative pressure produced in the suction chamber by the said vacuum or suction pump.

According to a another feature of the invention, the heat-insulated block and the measuring cell are provided with means adapted to hold them at one same constant temperature throughout the density determination.

According to a further feature of the invention, the walls of the suction chamber are transparent at least in part.

## Brief Description of the Drawings

One embodiment of the device of the invention is shown in the drawings as a non-limiting example thereof:

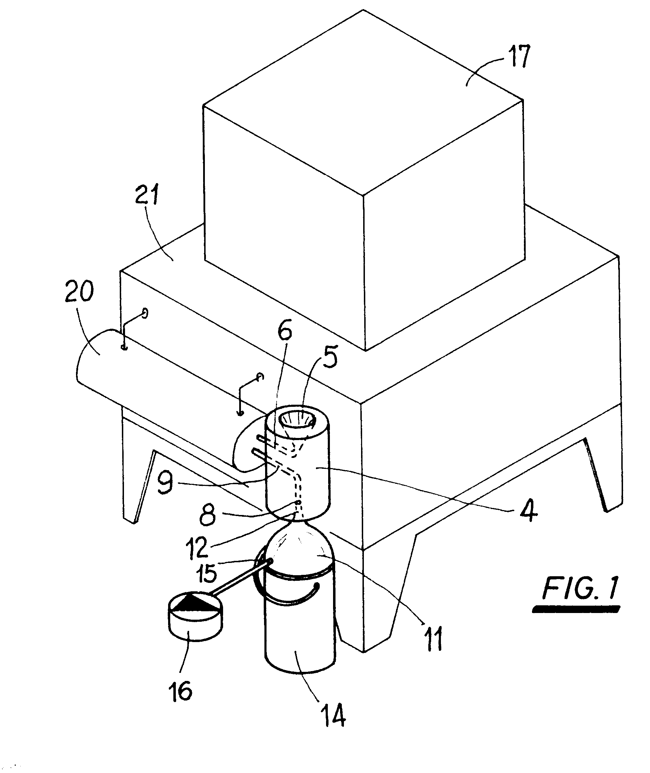

Figure 1 is a perspective view of a vibrating tube electronic densimeter, with the device of the invention attached thereto;

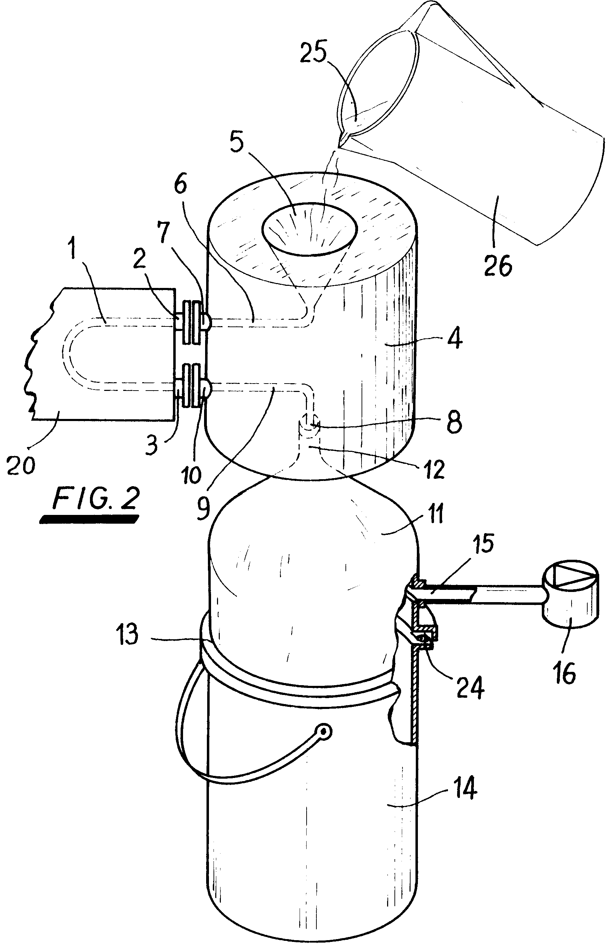

Figure 2 is a detail view also in perspective on a larger scale of the device in question; and

Figure 3 is a block diagram of the densimeter and feed device assembly.

In the drawings the electronic densimeter schematically illustrated therein is shown to comprise a measuring cell 1, provided with a charge port 2 and a discharge port 3 for the sample whose density is to be ascertained. The cell 1 is subject to constant vibration, the frequency of which varies in terms of the density of the sample introduced therein.

The device for feeding the samples into the cell 1 and which is the object of the invention comprises a heat-insulated block 4, provided with an upper sample inlet port 5, communicating over a first internal passage 6 with a charge port 7 and a lower sample outlet port, communicating over a second internal passage with a discharge port 10.

The charge and discharge ports 2 and 3 of the heat-insulated block 4 are so disposed that, in the operative position of the feed device, they are respectively firmly engaged face to face with the corresponding charge and discharge ports 7 and 10 of the cell 1.

Below the block 4 there is disposed a suction chamber 11, provided with an upper inlet port 12 attached to the lower outlet 8 of the block 4 by way of a simple device, such as a cork or rubber plug for example, and also provided with a lower opening 13 adapted to be sealingly attached, for example by way of a sealing gasket 24 to the open end of a waste collector container 14, into which the sample falls once the density thereof hes been determined.

The suction chamber 11 which is transparent, at least in part with a view to monitoring the correct introduction of the sample and the level to which the waste container has been filled, is also provided with a suction port 15, adapted to be connected to vacuum or suction means 16.

Thus, the sample is sucked into the measuring cell 1 by the gentle negative pressure created in the suction chamber 11 by the said vacuum or suction means 16.

The range of vacuum applied will extend from a gentle negative pressure, sufficient for the product to flow, up to a pressure of 1 mm Hg, or to the lower minimum pressure not damaging the measuring cell and not sucking air in.

The heat-insulated block 4 and the measuring cell 1 are provided with means adapted to hold them at one same constant temperature throughout the whole of the density determination. Said temperature is, preferably, 135°C, for bitumens and 70°C for viscous residues, although the temperature range at which the feed device of the invention may be used is broader and ranges approximately from 50°C to 150°C.

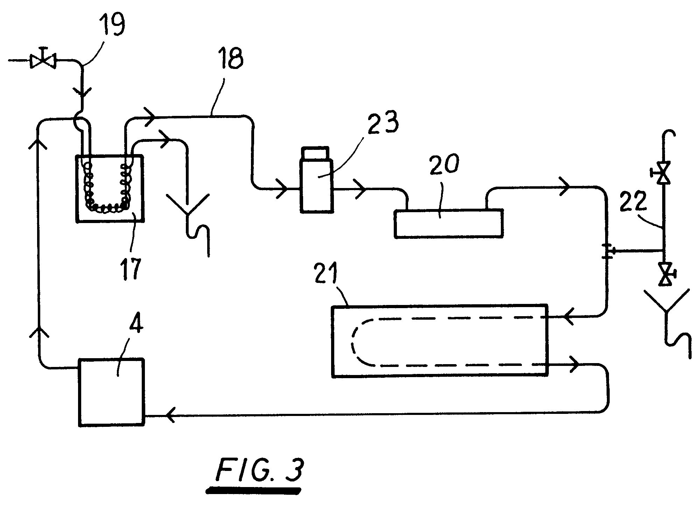

Figure 3 is a schematic block diagram of the joint assembly of adensimeterand the feed device of the invention. There is observed therein a thermostatic oil bath 17, capable of regulating the temperature to an accuracy of at least 0.01°C and a resolution of at least 0.1°C. The oil from bath 17 flows through the circuit 18, through the body member 20 enclosing the cell 1, through the cell 1 support block 21, completely through the heat-insulated block 4 and back to the bath 17.

The two joints between the body member 20 enclosing the cell 1 and the thermostatic circuit 18 are formed by respective short tubular portions of a flexible heat-resistant material, such as a silicone tube.

The said thermostatic bath 17 also comprises a purge circuit 22, an external control thermocouple 23 and a cooling liquid circuit 19 with which the oil of bath 17 is quickly cooled, for example if, immediately after determining the density of a bitumen the bath temperature is to be lowered to determine that of a viscous residue.

The operational steps for density determinations using a densimeter and an applicator device as described consists basically of raising the whole system to the required service temperature, pouring the sample 25 contained in a container 26, at a temperature practically the same as the said service temperature, in the upper inlet port 5 of the block 4, applying thereafter a gentle negative pressure at the port 15 by the means 16 until the amount of sample deemed necessary for correct cleaning of the circuit has entered in the chamber 11 through the port 12, at which time suction is withdrawn, the measurement is made and, finally, the whole sample 25 poured in the port 5 is aspirated again and, as necessary, the cell 1 and the rest of the passage are flushed with an appropriate product, also sucked in by the means 16. When the process or processes are terminated and the container 14 is full, it is disposed of with the contents.

Obviously, if it is undesirable to dispose of the sample, it may be led directly to a recovery cycle.

Having sufficiently described the nature of the invention and the way of reducing it to practice, it is affirmed that all that does not alter, change or modify the fundamental principle thereof, may be subject to variations of detail, the essence, for which a patent for twenty years is petitioned, being as summarised in the following claims.

**Claims**

1.- A sample feed device for vibrating tube electronic densimeters, of the type particularly applicable to the determination of the density of high viscosity materials, such as bituminous materials, and comprise a measuring cell (1), provided with a charge port (2) and a discharge port (3) for the sample whose density is to be determined, said measuring cell being subjected to constant vibration, the frequency of which varies in function of the density of the sample introduced in the cell, characterized in that it comprises a heat-insulated block (4) provided with an upper sample inlet port (5), communicating over a first internal passage (6) with a charge port (7) and a lower sample outlet port (8), communicating with a discharge port (10) over a second internal passage (9), said charge (7) and discharge (10) ports of the heat-insulated block being so disposed that in the operative position of the feed device they are respectively firmly engaged with the corresponding charge (2) and discharge (3) ports of the measuring cell, there being disposed below the block a suction chamber (11) provided with an upper inlet port (12), connected to the lower outlet port (8) of the block, having a lower mouth (13) adapted sealingly to be attached to the mouth of a waste collector container (14) in which the sample falls after the density thereof has been determined, and a suction port (15), adapted to be connected to a vacuum or suction means (16), the arrangement being such that the sample is introduced in the measuring cell by suction thereof, due to the negative pressure produced in the suction chamber by the said vacuum or suction means.

2.- The sample feed device for densimeters of claim 1, characterised in that the heat-insulated block (4) and the measuring cell (1) are provided with means adapted to hold them at one same constant temperature throughout the density determination.

3.- The sample feed device for densimeters of claims 1 and 2, characterised in that the walls of said suction chamber (11) are transparent at least in part.

*FIG. 1*

*FIG. 2*

FIG. 3